(19) Europäisches Patentamt
European Patent Office
Office européen des brevets

(11) EP 1 419 763 B1

(12) # FASCICULE DE BREVET EUROPEEN

(45) Date de publication et mention de la délivrance du brevet:
**26.07.2006 Bulletin 2006/30**

(21) Numéro de dépôt: **03292591.9**

(22) Date de dépôt: **17.10.2003**

(51) Int Cl.:
***A61K 8/81*** *(2006.01)* ***A61K 8/85*** *(2006.01)*
***A61K 8/86*** *(2006.01)* ***A61K 8/87*** *(2006.01)*
***A61K 8/899*** *(2006.01)* ***A61Q 19/00*** *(2006.01)*

(54) **Composition cosmétique comprenant un latex tenseur et un polymère amphiphile ionique**

Kosmetische Zusammensetzung enthaltend ein Spannhilfsstofflatex und ein amphiphiles ionisches Polymer

Cosmetic composition comprising tensioning latex and an amphiphilic ionic polymer

(84) Etats contractants désignés:
**AT BE BG CH CY CZ DE DK EE ES FI FR GB GR HU IE IT LI LU MC NL PT RO SE SI SK TR**

(30) Priorité: **12.11.2002 FR 0214116**

(43) Date de publication de la demande:
**19.05.2004 Bulletin 2004/21**

(73) Titulaire: **L'ORÉAL**
**75008 Paris (FR)**

(72) Inventeur: **Cassin, Guillaume**
**91140 Villebon sur Yvette (FR)**

(74) Mandataire: **Kromer, Christophe**
**L'OREAL - D.I.P.I.**
**25-29 Quai Aulagnier**
**92600 Asnières (FR)**

(56) Documents cités:
**EP-A- 1 043 385**
**EP-A- 1 046 387**
**EP-A- 1 129 684**
**WO-A-98/29091**
**WO-A-98/29092**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 419 763 B1

## Description

La présente invention se rapporte à une composition adaptée à une application topique sur la peau et renfermant une phase aqueuse, une phase grasse et une dispersion de particules de polymère produisant, à une concentration de 7% en poids dans l'eau, une rétraction du *stratum corneum* isolé de plus de 1,5% à 30°C et sous une humidité relative de 40%, caractérisée en ce que ladite composition renferme en outre au moins un polymère amphiphile ionique, et moins de 1% en poids, par rapport au poids total de la composition, de tensioactif différent dudit polymère amphiphile ionique.

Au cours du processus de vieillissement, il apparaît différents signes caractéristiques sur la peau, résultant notamment d'une modification de la structure et des fonctions cutanées. Les principaux signes cliniques du vieillissement cutané sont notamment l'apparition de ridules et de rides profondes, en augmentation avec l'âge. On constate en particulier une désorganisation du "grain" de la peau, c'est-à-dire que le micro-relief est moins régulier et présente un caractère anisotrope.

Il est connu de traiter ces signes du vieillissement en utilisant des compositions cosmétiques ou dermatologiques contenant des actifs capables de lutter contre le vieillissement, tels que les $\alpha$-hydroxy-acides, les $\beta$-hydroxy-acides et les rétinoïdes. Ces actifs agissent sur les rides en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat des actifs utilisés.

A cette fin, il a été proposé depuis quelques années des agents à effet tenseur qui lissent immédiatement après application les rides et ridules et contribuent à atténuer les marques de fatigue. Ces composés agissent en formant un film provoquant la rétraction du *stratum corneum,* la couche cornée superficielle de l'épiderme. Des exemples de tels agents tenseurs sont notamment des dispersions aqueuses de polymères synthétiques (WO 98/29092), en particulier de polymères en "étoile" (EP-1 043 345), de polymère siliconé greffé (EP-1 038 519) ou de polymère comportant des unités à LCST.

Toutefois, ces agents tenseurs de l'art antérieur sont rarement formulés en présence d'huile, en particulier dans des émulsions H/E ou E/H, mais plus souvent sous forme de sérums, c'est-à-dire de compositions aqueuses gélifiées. Il est en effet généralement observé que l'efficacité de ces composés était grandement diminuée en présence d'huile.

Or, la présence d'une phase huileuse dans une composition cosmétique est souvent souhaitable, que ce soit pour conférer un caractère émollient à la composition ou pour véhiculer divers actifs topiques liposolubles, tels que les vitamines A et E, notamment, qui sont particulièrement utiles dans des compositions anti-rides.

Il reste donc le besoin de disposer d'une composition à effet tenseur, contenant une phase aqueuse, une phase huileuse et une dispersion de particules de polymère tenseur, qui soit stable dans le temps en vue d'une utilisation commerciale et qui offre des propriétés de tension de la peau suffisantes pour lisser visiblement les rides et ridules dès son application.

Or, la Demanderesse a découvert avec étonnement que la diminution du pouvoir tenseur des latex lorsqu'ils sont formulés en émulsion était due à la présence de tensioactifs dans ces émulsions et qu'en substituant ces tensioactifs, en tout ou partie, par un ou plusieurs polymère(s) amphiphile(s) ionique(s), il était possible d'obtenir une émulsion ayant un meilleur effet tenseur que celles de l'art antérieur, sans perte de stabilité de l'émulsion.

L'invention a donc pour objet une composition adaptée à une application topique sur la peau et renfermant une phase aqueuse, une phase grasse et une dispersion de particules de polymère produisant, à une concentration de 7% en poids dans l'eau, une rétraction du *stratum corneum* isolé de plus de 1,5% à 30°C et sous une humidité relative de 40%, caractérisée en ce que ladite composition renferme en outre au moins un polymère amphiphile ionique, et moins de 1% en poids, par rapport au poids total de la composition, de tensioactif différent dudit polymère amphiphile ionique.

Elle a également pour objet un procédé de traitement cosmétique d'une peau ridée, comprenant l'application sur la peau de la composition décrite précédemment.

Elle a encore pour objet l'utilisation cosmétique de la composition décrite précédemment pour lisser ou atténuer les rides et ridules et/ou retendre la peau.

La dispersion de particules de polymère utilisée selon l'invention, encore appelée "latex", est de préférence constituée d'un polymère synthétique et peut être constituée de polymères et copolymères de polyuréthanne, en particulier de copolymères polyester-polyuréthanne ou de copolymères polyéther-polyuréthanne ; de polymères et copolymères acryliques ; ou de polymères d'acide isophtalique sulfoné.

Ces polymères peuvent se trouver sous la forme de polycondensats, de polymères hybrides et de réseaux de polymères interpénétrés (IPNs).

Par "réseau de polymères interpénétrés" au sens de la présente invention, on entend un mélange de deux polymères enchevêtrés, obtenu par polymérisation et/ou réticulation simultanée de deux types de monomères, le mélange obtenu ayant une température de transition vitreuse unique.

Des exemples d'IPNs convenant à une mise en oeuvre dans la présente invention, ainsi que leur procédé de préparation, sont décrits dans les brevets US-6,139,322 et US-6,465,001, par exemple.

De préférence, l'IPN selon l'invention comprend au moins un polymère polyacrylique et, plus préférentiellement, il comprend en outre au moins un polyuréthane ou un copolymère de fluorure de vinylidène et d'hexafluoropropylène.

Selon une forme d'exécution préférée, l'IPN selon l'invention comprend un polymère polyuréthane et un polymère polyacrylique. De tels IPNs sont notamment ceux de la série Hybridur qui sont disponibles dans le commerce auprès de la société AIR PRODUCTS.

Un IPN particulièrement préféré se trouve sous la forme d'une dispersion aqueuse de particules ayant une taille moyenne, en poids, comprise entre 90 et 110 nm et une taille moyenne, en nombre, d'environ 80 nm. Cet IPN a de préférence une température de transition vitreuse, Tg, qui va d'environ -60°C à +100°C. Un IPN de ce type est notamment commercialisé par la société AIR PRODUCTS sous la dénomination commerciale Hybridur X-01602. Un autre IPN convenant à une utilisation dans la présente invention est référencé Hybridur X18693-21.

D'autres IPNs convenant à une mise en oeuvre dans la présente invention comprennent les IPNs constitués du mélange d'un polyuréthane avec un copolymère de fluorure de vinylidène et d'hexafluoropropylène. Ces IPNs peuvent notamment être préparés comme décrit dans le brevet US-5,349,003. En variante, ils sont disponibles dans le commerce sous forme de dispersion colloïdale dans l'eau, dans un rapport du copolymère fluoré au polymère acrylique comprise entre 70:30 et 75:25, sous les dénominations commerciales KYNAR RC-10,147 et KYNAR RC-10,151 auprès de la société ATOFINA.

Des polymères sous forme de polycondensats ont notamment été décrits dans la demande (WO 98/29092). Ils comprennent en particulier ceux commercialisés sous les dénominations commerciales AVALURE UR410, AVALURE UR460 par la société NOVEON, et sous les dénominations commerciales NEOREZ R974, NEOREZ R981 et NEOREZ R970 par la société AVECIA, ainsi que le copolymère NEOCRYL XK-90 commercialisé par la société AVECIA.

La dispersion de particules de polymère utilisée selon l'invention peut en variante être constituée d'un polymère siliconé greffé, tel que défini dans la demande EP-1 038 519, et plus particulièrement un polymère comprenant une chaîne principale de silicone (ou polysiloxane ($(\equiv Si\text{-}O\text{-})_n$) sur laquelle se trouve greffé, à l'intérieur de ladite chaîne ainsi qu'éventuellement à l'une au moins de ses extrémités, au moins un groupement organique ne comportant pas de silicone.

Un exemple préféré de polymère siliconé greffé est le polysilicone-8 (nom CTFA) qui est un polydiméthylsiloxane sur lequel sont greffés, par l'intermédiaire d'un chaînon de raccordement de type thiopropylène, des motifs polymères mixtes du type acide poly(méth)acrylique et du type poly(méth)acrylate d'alkyle. Un polymère de ce type est notamment disponible sous la dénomination commerciale VS 80 (à 10% dans l'eau) ou LO 21 (sous forme pulvérulente) auprès de la société 3M. Il s'agit d'un copolymère de polydiméthylsiloxane à groupements propylthio, d'acrylate de méthyle, de méthacrylate de méthyle et d'acide méthacrylique.

Selon une autre possibilité encore, le latex utilisé selon l'invention peut être constitué d'un polymère de structure en "étoiles" représentée par la formule suivante (I) :

$$A\text{-}[(M1)_{p1}\text{-}(M2)_{p2} \ldots (Mi)_{pj}]_n \qquad (I)$$

dans laquelle :

- A représente un centre multifonctionnel, de fonctionnalité "n", n étant un entier supérieur à 2,
- $[(M1)_{p1}\text{ - }(M2)_{p2} \ldots (Mi)_{pj}]$ représente une chaîne polymérique, aussi appelée "branche", constituée de monomères Mi polymérisés, identiques ou différents, ayant un indice de polymérisation pj, chaque branche étant identique ou différente, et étant greffée de manière covalente sur ledit centre A,
- i étant supérieur ou égal à 1, et pj étant supérieur ou égal à 2;

ledit polymère comprenant un ou plusieurs monomères Mi dont l'homopolymère correspondant présente une Tg supérieure ou égale à environ 10°C, de préférence supérieure ou égale à 15°C, et encore mieux supérieure ou égale à 20°C;

- ce ou ces monomères Mi étant présents, dans le polymère final, en une quantité minimale d'environ 45% en poids, de préférence en une quantité comprise entre 55 et 99% en poids, et encore mieux en une quantité de 75-90% en poids, par rapport au poids total de monomères.

Ces polymères, ainsi que leur procédé de préparation, sont décrits dans la demande EP-1 043 345.

Les latex ou dispersions de particules de polymère utilisables dans la présente invention ne sont pas limitées aux types précités mais peuvent être constituées de tout type de polymère ayant un effet tenseur suffisant, caractérisé par la valeur de rétraction du *stratum corneum* définie ci-dessus. Cette valeur de rétraction est mesurée à l'extensomètre, selon la méthode décrite dans l'Exemple 5 ci-après.

La quantité de polymère tenseur présente dans la composition peut varier dans une large mesure en fonction

de l'effet recherché. A titre d'exemple, le polymère tenseur peut représenter de 0,1 à 50% en poids, et de préférence de 1 à 20% en poids, par rapport au poids total de la composition.

Comme indiqué précédemment, la composition selon l'invention renferme également un polymère amphiphile ionique.

Par cette expression, on entend des polymères cationiques, anioniques ou amphotères comportant à la fois une partie hydrophile et une partie hydrophobe et ayant la propriété de former un film séparant deux liquides de polarité différente et permettant ainsi de stabiliser des dispersions liquide - liquide de type direct, inverse ou multiple. Plus précisément, les polymères amphiphiles selon l'invention ont la propriété de réduire la tension interfaciale eau/ huile jusqu'à 10 mN/m, et ce quelque soit l'huile. Ils peuvent être hydrosolubles ou hydrodispersibles. On entend par hydrosoluble le fait qu'ils puissent se disperser dans l'eau sous forme de solution moléculaire. On entend par hydrodispersible le fait qu'ils puissent se disperser dans l'eau sous forme particulaire.

Les polymères amphiphiles conformes à l'invention ont généralement un poids moléculaire moyen en nombre allant de 1000 à 20 000 000 g/mol, de préférence allant de 20 000 à 8 000 000 g/mol et plus préférentiellement encore de 100 000 à 700 000 g/mol.

De façon préférentielle, les polymères amphiphiles ioniques utilisés selon l'invention ne forment pas de films cohésifs au séchage. On entend par formation d'un film cohésif le fait qu'après déposition sur une plaque de verre et évaporation du solvant soit formé un film macroscopique que l'on peut décoller de la plaque de verre et manipuler.

Des exemples de polymères amphiphiles ioniques convenant à une mise en oeuvre dans la présente invention seront maintenant décrits plus en détail.

A-Polymères amphiphiles ioniques hydrosolubles

A-1- Copolymères acryliques

Les copolymères acryliques utilisables comme polymères amphiphiles ioniques selon l'invention sont des composés obtenus par copolymérisation d'un monomère (a) choisi parmi les acides carboxyliques à insaturation $\alpha,\beta$-éthylénique avec un monomère (b) issu de la réaction d'un monomère acide carboxylique à insaturation éthylénique avec un alcool gras aliphatique éventuellement alcoxylé, en particulier polyéthoxylé, dont la chaîne carbonée renferme au moins 6 atomes de carbone.

Selon une première variante, ces copolymères acryliques peuvent être choisis parmi les copolymères, éventuellement réticulés et/ou neutralisés, constitués d'une fraction majoritaire de monomère acide carboxylique mono-oléfiniquement insaturé en $C_3$-$C_6$ ou de son anhydride et d'une fraction minoritaire de monomère ester à chaîne grasse d'acide acrylique. Ces copolymères sont préparés en polymérisant une quantité prépondérante de monomère acide carboxylique monooléfiniquement insaturé ou de son anhydride, à une quantité plus faible de monomère ester acrylique à chaîne grasse. On entend par chaîne grasse un radical alkyle linéaire ou ramifié, comportant de 8 à 30 atomes de carbone.

La quantité de monomère acide carboxylique ou de son anhydride va de préférence de 80 à 98 % en poids et plus particulièrement de 90 à 98 % en poids, tandis que l'ester acrylique est présent dans des quantités allant de 2 à 20 % en poids et plus particulièrement de 1 à 10 % en poids, les pourcentages étant calculés par rapport au poids total des deux monomères.

Les monomères carboxyliques préférentiels sont choisis parmi ceux répondant à la formule (II) suivante :

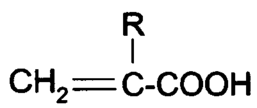

(II)

où R désigne l'hydrogène, un halogène, un groupe ,hydroxyle, un groupe lactone, un groupe lactame, un groupe cyanogène (-C=N), un groupe alkyle monovalent, un groupe aryle, un groupe alkylaryle, un groupe aralkyle ou un groupe cycloaliphatique.

Les monomères esters acryliques à chaîne grasse sont préférentiellement choisis parmi ceux répondant à la formule (III) suivante :

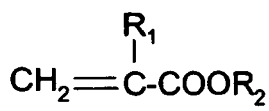

(III)

où $R_1$ est choisi dans le groupe formé par l'hydrogène, le radical méthyle et le radical éthyle, et $R_2$ est un groupe alkyle en $C_8$-$C_{30}$.

On utilise plus particulièrement les polymères formés à partir d'un mélange de monomères comprenant de l'acide acrylique et un ester de formule (III) dans laquelle R1 désigne H ou CH3, R2 désignant un radical alkyle ayant de 12 à 22 atomes de carbone, et un agent réticulant.

Les copolymères acryliques préférés pour une utilisation dans la présente invention sont constitués de 95 à 60% en poids d'acide acrylique, 4 à 40% en poids d'acrylate d'alkyles en C10-C30, et 0 à 6% en poids de monomère polymérisable réticulant, ou 98 à 96% en poids d'acide acrylique, 1 à 4% en poids d'acrylate d'alkyles en C10-C30, et 0,1 à 0,6% en poids de monomère polymérisable réticulant.

L'agent réticulant peut être utilisé en une quantité allant de 0,1 à 4 %, de préférence de 0,2 à 1 % en poids par rapport au poids total de monomères carboxyliques et de monomères esters acryliques. L'agent réticulant peut être choisi notamment parmi les monomères polymérisables contenant un groupe $CH_2$=C- polymérisable et au moins un autre groupe polymérisable, dont les liaisons insaturées ne sont pas conjuguées l'une par rapport à l'autre. Un monomère polymérisable réticulant préféré pour une utilisation dans la présente invention est choisi parmi : le polyallylsucrose et le polyallylpentaérythritol.

Ces copolymères sont décrits dans le document EP-A-0268164 et sont obtenus selon les méthodes de préparation décrites dans ce même document.

De façon préférentielle, les polymères conformes à l'invention sont neutralisés partiellement ou totalement par une base minérale (par exemple soude, potasse, ammoniaque) ou une base organique telle que la mono-, di- et tri-éthanolamine, l'aminométhylpropanediol, la N- méthyl-glucamine, les acides aminés basiques comme l'arginine et la lysine, et leurs mélanges.

Des exemples préférés de copolymères acryliques utilisables dans la présente invention sont les copolymères acrylate/$C_{10}$-$C_{30}$-alkylacrylate (nom CTFA) vendus sous les noms PEMULEN TR1, PEMULEN TR2 et CARBOBOL 1382 par la Société GOODRICH, ou bien leurs mélanges.

Selon une seconde variante, le copolymère acrylique utilisable comme polymère amphiphile ionique selon la présente invention peut être obtenu par copolymérisation d'un monomère (a) choisi parmi les acides carboxyliques à insaturation $\alpha,\beta$-éthylénique avec un monomère (b) issu de la réaction de l'acide itaconique avec un alcool gras aliphatique alcoxylé, en particulier polyéthoxylé, comprenant de 1 à 50 motifs oxyde d'alkylène, dont la chaîne carbonée renferme au moins 6 atomes de carbone.

Un tel copolymère peut être un copolymère d'acide acrylique et d'itaconate de mono-stéaryle oxyéthyléné (20 OE) ou un copolymère d'acide acrylique et d'itaconate de mono-cétyle oxyéthyléné (20 OE). De tels copolymères sont vendus sous les noms STRUCTURE 2001 et STRUCTURE 3001 par la Société NATIONAL STARCH.

A-2- Dérivés d'AMPS hydrophobés

Les polymères amphiphiles ioniques selon l'invention peuvent être, entres autres, des polymères amphiphiles comportant au moins un monomère à insaturation éthylénique à groupement sulfonique, sous forme libre ou partiellement ou totalement neutralisée et comprenant au moins une partie hydrophobe. La partie hydrophobe présente dans ces polymères comporte de préférence de 6 à 50 atomes de carbone, plus préférentiellement de 6 à 22 atomes de carbone, plus préférentiellement encore de 6 à 18 atomes de carbone, et plus particulièrement de 12 à 18 atomes de carbone.

Ces polymères peuvent être des polymères non réticulés à base d'au moins un monomère hydrophile A à insaturation éthylénique et d'au moins un monomère hydrophobe B. De préférence, le monomère A comporte une fonction acide fort, en particulier, une fonction acide sulfonique ou acide phosphonique. Le monomère hydrophobe B comporte au moins un radical hydrophobe, choisi parmi :les radicaux alkyles linéaires en $C_6$-$C_{18}$ , saturés ou insaturés (par exemple n-hexyle, n-octyle, n-décyle, n-hexadécyle, n-dodécyle, oléyle), ramifiés (par exemple isostéarique) ou cycliques (par exemple cyclododécane ou adamantane), les radicaux fluorés ou alkylfluorés en $C_6$-$C_{18}$ (par exemple le groupement de formule $-(CH_2)_2-(CF_2)_9-CF_3$), le radical cholestéryle ou les radicaux dérivés de cholestérol (par exemple l'hexanoate de cholestéryle), les groupes polycycliques aromatiques comme le naphtalène ou le pyrène, les radicaux siliconés ou alkylsiliconés ou encore alkylfluorosiliconés. Parmi ces radicaux, on préfère plus particulièrement les radicaux alkyles linéaires et ramifiés.

Ces polymères sont solubles ou dispersibles dans l'eau, sous forme neutralisée. Leur viscosité à une concentration de 1% dans l'eau, à un gradient de cisaillement de $1s^{-1}$, à un pH compris entre 5 et 8, à 25°C, est inférieure à 5000mPas. Les polymères de l'invention peuvent notamment être choisis parmi les polymères amphiphiles non réticulés décrits dans les documents EP1138703 et EP1069142.

Les polymères amphiphiles selon l'invention peuvent aussi être réticulés. Les agents de réticulation peuvent être choisis parmi les composés à polyinsaturation oléfinique couramment utilisés pour la réticulation des polymères obtenus par polymérisation radicalaire. Selon un mode préféré de réalisation de l'invention, l'agent de réticulation est choisi parmi le méthylène-bis-acrylamide, le méthacrylate d'allyle ou le triméthylol propane triacrylate (TMPTA). Le taux

de réticulation varie en général de 0,01 à 10% en mole et plus particulièrement de 0,2 à 2% en mole par rapport au polymère.

Les polymères préférentiels de l'invention sont choisis parmi les polymères amphiphiles réticulés ou non d'acide 2-acrylamido 2-méthylpropane sulfonique (AMPS) et d'au moins un monomère à insaturation éthylénique comportant au moins une partie hydrophobe ayant de 6 à 30 atomes de carbone et plus préférentiellement de 6 à 22 atomes de carbone et encore plus préférentiellement de 6 à 18 atomes de carbone et plus particulièrement de 12 à 18 atomes de carbone.

On citera plus particulièrement les copolymères constitués :

(a) de 40 à 99% en moles de motif acide 2-acrylamido 2-méthylpropane sulfonique (AMPS) de formule (IV) suivante :

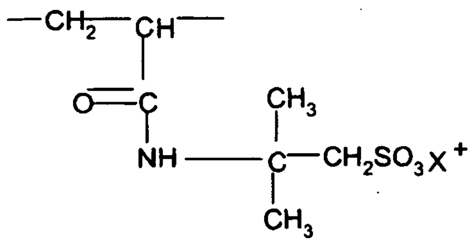

(IV)

dans laquelle $X^+$ est un proton, un cation de métal alcalin, un cation alcalino-terreux ou l'ion ammonium ; et
(b) de 1 à 60% en moles de motif de formule (V) suivante :

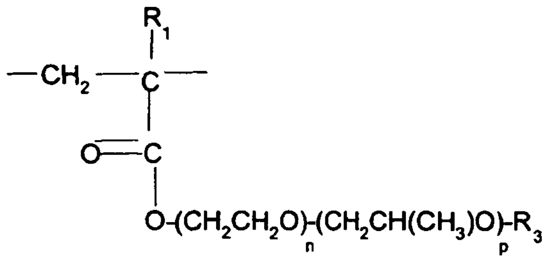

(V)

dans laquelle n et p, indépendamment l'un de l'autre désignent un nombre de moles et varie de 0 à 30, de préférence de 1 à 25 et plus préférentiellement de 3 à 20 sous réserve que n + p soit inférieur ou égal à 30, de préférence inférieur à 25 et encore mieux inférieur à 20 ; $R_1$ désigne un atome d'hydrogène ou un radical alkyle linéaire ou ramifié en $C_1$-$C_6$ (de préférence méthyle) et $R_3$ désigne un alkyle linéaire ou ramifié comportant de 6 à 30 atomes de carbone, de préférence de 6 à 22 atomes de carbone et encore mieux de 12 à 18 atomes de carbone.

De façon préférentielle, les polymères conformes à l'invention sont neutralisés partiellement ou totalement par une base minérale (par exemple soude, potasse, ammoniaque) ou une base organique telle que la mono-, di- et tri-éthanolamine, l'aminométhylpropanediol, la N- méthyl-glucamine, les acides aminés basiques comme l'arginine et la lysine, et leurs mélanges.

Parmi les polymères amphiphiles réticulés ou non d'AMPS, on utilisera plus particulièrement les produits vendus sous les noms ARISTOFLEX HMS et E-47/2000W1 par la Société CLARIANT.

A-3- Terpolymères acryliques

Les polymères amphiphiles ioniques selon l'invention peuvent aussi être choisis parmi les terpolymères acryliques obtenus à partir de (a) un acide carboxylique à insaturation $\alpha,\alpha$-éthylénique, (b) un monomère à insaturation éthylénique non tensioactif différent de (a), et (c) un monomère uréthanne non-ionique qui est le produit de réaction d'un composé amphiphile non-ionique monohydrique avec un isocyanate à insaturation monoéthylénique. Le terpolymère acrylique défini ci-dessus peut être obtenu par copolymérisation en dispersion aqueuse des composants (a), (b) et (c), copolymérisation qui est tout à fait usuelle et décrite notamment dans le document EP-A-0 173 109.

De façon préférentielle, ces polymères amphiphiles sont neutralisés partiellement ou totalement par une base minérale (soude, potasse, ammoniaque) ou une base organique telle que la mono-, di- ou tri-éthanolamine, l'aminométhylpropanediol, la N-méthyl-glucamine, les acides aminés basiques comme l'arginine et la lysine, et les mélanges de ces composés.

A titre de terpolymères pouvant être utilisés selon l'invention, on peut citer le terpolymère acide méthacrylique/acrylate de méthyle/diméthyl m-isopropénylbenzylisocyanate d'alcool béhénylique éthoxylé à 40 OE, c'est-à-dire comportant 40 groupes oxyéthylénés, vendu par la société AMERCHOL sous les dénominations VISCOPHOBE DB 1000 NP3 et NP4.

D'autres terpolymères acryliques utilisables dans la présente invention comprennent les polymères amphiphiles dont le motif hydrophile est constitué par un monomère anionique insaturé éthylénique, plus particulièrement par un acide carboxylique vinylique et tout particulièrement par un acide acrylique, un acide méthacrylique ou leurs mélanges, et dont le motif hydrophobe est un éther d'allyle à chaîne grasse correspondant au monomère de formule (VI) suivante :

CH2 = C(R') CH2 O Bn R (VI)

dans laquelle R' désigne H ou $CH_3$, B désigne le radical éthylèneoxy, n est nul ou désigne un entier allant de 1 à 100, R désigne un radical hydrocarboné choisi parmi les radicaux alkyl, arylalkyl, aryl, alkylaryl, cycloalkyl, comprenant 8 à 30 atomes de carbone, de préférence 10 à 24, et plus particulièrement encore de 12 à 18 atomes de carbone. Un motif de formule (VI) plus particulièrement préféré est un motif dans lequel R' désigne H, n est égal à 10, et R désigne un radical stéaryle ($C_{18}$). Des polymères amphiphiles anioniques de ce type sont décrits et préparés, selon un procédé de polymérisation en émulsion, dans le brevet EP-0 216 479.

Parmi ces polymères amphiphiles anioniques, on préfère particulièrement selon l'invention, les polymères formés à partir de 20 à 60% en poids d'acide acrylique et/ou d'acide méthacrylique, de 5 à 60% en poids de (méth)acrylates d'alkyle inférieurs, de 2 à 50% en poids d'éther d'allyle à chaîne grasse de formule (VI), et de 0 à 1% en poids d'un agent réticulant qui est un monomère insaturé polyéthylénique copolymérisable bien connu, comme le phtalate de diallyle, le (méth)acrylate d'allyle, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, d'allyléther de polyéthylèneglycol (10 OE) éther d'alcool stéarylique (40/50/10), notamment ceux vendus par la société ALLIED COLLOIDS sous les dénominations SALCARE SC 80 et SALCARE SC90 sous forme d'émulsions aqueuses à 30%.

B-Polymères amphiphiles ioniques hydrodispersibles

Les polymères amphiphiles utilisables selon l'invention peuvent être, en variante, hydrodispersibles.

Les polymères anioniques amphiphiles hydrodispersibles utilisables selon l'invention sont par exemple des copolyesters sulfo-isophtaliques consistant essentiellement en des unités répétées d'acide isophtalique (ou d'esters ou de chlorure d'acide isophtalique), de diol et d'acide sulfo-isophtalique.

Le diol présent dans le copolyester sulfo-isophtalique peut être un diol cycloaliphatique ayant de préférence de 6 à 20 atomes de carbone ou un diol aliphatique ayant de préférence de 3 à 20 atomes de carbone. Comme diol, on peut utiliser l'éthylène glycol, le diéthylène glycol, le triéthylène glycol, le 1,4-cyclohexanediméthanol, le propane -1,3-diol, le butane-1,4-diol, le pentane-1,5-diol, l'hexane-1,6-diol, le 3-méthylpentanediol-(2,4), le 2-méthylpentanediol-(1,4), le 2,2,4-triméthylpentanediol-(1,3), le 2-éthylhexanediol-(1,3), le 2,2-diéthylpropane-diol-(1,3), l'hexanediol-(1,3), le 1,4-di-(hydroxyéthoxy)-benzène, le 2,2-bis-(4-hydroxycyclohexyl)-propane, le 2,4-dihydroxy-1,1,3,3-tétraméthyl-cyclobutane, le 2,2-bis-(3-hydroxyéthoxyphényl)-propane, le 2,2-bis-(4-hydroxypropoxyphényl)-propane. Le copolyester isophtalique peut comporter un ou plusieurs diols tels que cités précédemment et de préférence un mélange d'éthylène glycol ou de 1,4-cyclohexanediméthanol et de diéthylène glycol. Avantageusement, le copolyester sulfo-isophtalique peut comporter un mélange de diol consistant en au moins 45 % en mole de diéthylène glycol et en complément à 100 % en mole d'éthylène glycol ou de 1,4-cyclohexane diméthanol.

Le motif acide sulfo-isophtalique comporte un groupement -SO3M avec M représentant un atome d'hydrogène, un ion ammonium NH4+ ou un ion métallique , comme par exemple Na+, Li+, K+, M étant de préférence Na+. L'acide sulfo-isophtalique particulièrement préféré est le sel de sodium de l'acide 5-sulfo-isophtalique.

Le motif acide sulfo-isophtalique est de préférence présent dans le copolyester sulfo-isophtalique en une teneur pouvant aller de 4 à 25 % en moles pour 100 moles d'acides et 100 moles de diols.

Avantageusement, le copolyester sulfo-isophtalique peut comprendre, pour 100 moles d'acides, de 75 % à 90 % en mole d'acide isophtalique et de 10 % à 25 % en mole d'acide sulpho-isophtalique, notamment de sel de sodium de l'acide 5-sulfoisophtalique, et, pour 100 moles de diols, de 45 % à 85 % en mole de diéthylène glycol et de 15 % à 55 % en mole de 1,4-cyclohexanediméthanol ou d'éthylène glycol ou leur mélange.

De tels copolyesters sulfo-isophtaliques sont notamment décrits dans les brevets US-A-3779993 et US-A-5260052 et sont par exemple commercialisés sous la dénomination "EASTMAN AQ 35S", " EASTMAN AQ 38S",

" EASTMAN AQ 55S", "EASTMAN AQ 29D", "EASTMAN AQ 38D" par la société EASTMAN CHEMICAL.

De façon avantageuse, les particules de polymère ionique utilisées selon l'invention ont une granulométrie allant de 10 à 400 nm et encore mieux allant de 20 à 200 nm selon la nature du polymère ionique. Les particules de ces polymères peuvent être utilisées telles quelles ou en dispersion dans l'eau. Le rapport en poids des particules de polymère à la phase huileuse va avantageusement de 1/5 à 1/40, et de préférence de 1/10 à 1/20. Un tel rapport permet d'obtenir une dispersion d'huile fluide, fine, c'est-à-dire ayant des globules d'une granulométrie inférieure à 500 nm, et parfaitement stable.

La quantité de polymère amphiphile ionique utilisée dans la composition selon l'invention est de préférence comprise entre 0,01 et 20% en poids, mieux, entre 0,1 et 10% en poids, encore mieux, entre 0,2 et 5% en poids, par rapport au poids total de la composition.

Le polymère amphiphile ionique précité étant destiné à se substituer à tout ou partie des tensioactifs classiquement utilisés dans les émulsions cosmétiques, la quantité de tensioactif présent dans la composition selon l'invention est inférieure à 1% en poids et de préférence inférieure à 0,5% en poids, par rapport au poids total de la composition.

Par "tensioactif', on entend tout composé ou mélange de composés identifiés comme tels dans l'un au moins des deux ouvrages suivants : Mc Cutcheon's : Emulsifiers and Detergents, International Edition 1998, et International Cosmetic Ingredient Dictionary and Handbook (CTFA).

Le polymère amphiphile ionique permet la réalisation d'émulsions stables même en présence de grandes quantités d'eau. Par conséquent, selon une forme d'exécution avantageuse, la composition selon l'invention renferme au moins 45% en poids d'eau. En outre, la composition selon l'invention renferme généralement au moins 1% en poids de phase grasse.

Cette composition peut se présenter sous toutes les formes galéniques classiquement utilisées pour une application topique et notamment sous forme d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

Selon un mode préféré de réalisation de l'invention, la composition se présente sous forme d'une émulsion huile-dans-eau (H/E).

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules $R^1COOR^2$ et $R^1OR^2$ dans laquelle $R^1$ représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, l'isohexadecane, l'isododecane, la vaseline, les polydécènes, le polyisobutène hydrogéné tel que l'huile de Parléam®;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique,

liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Parmi les huiles indiquées ci-dessus, les huiles démaquillantes sont plus particulièrement les esters d'acide gras comportant au moins 12 atomes de carbone. Ces esters sont de préférence obtenus à partir d'un alcool à chaîne droite ou ramifiée, comportant de 1 à 17 atomes de carbone et d'un acide gras à chaîne droite ou ramifiée, comportant au moins 12 atomes de carbone et de préférence de 14 à 22 atomes de carbone. Il s'agit de préférence de mono- ou di-esters. Comme huile démaquillante, on peut citer par exemple le palmitate d'éthyl-2 hexyle (ou palmitate d'octyle), le myristate d'éthyl-2 hexyle (ou myristate d'octyle), le palmitate d'isopropyle, le myristate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, et leurs mélanges.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

L'invention sera maintenant illustrée par les exemples non limitatifs suivants. Dans ces exemples, les quantités sont indiquées en pourcentage pondéral.

## EXEMPLES

### Exemple 1 (comparatif) : Composition cosmétique

| | |
|---|---|
| *Phase A* | |
| Tartrate de dimyristyle, alcool cétylstéarylique, alcool laurylique oxyéthyléné (25 OE) oxypropyléné (25 OP) et alcools gras en $C_{12-15}$ oxyéthylénés (7 OE) (COSMACOL PSE de SASOL) | 1,50 g |
| Stéarate de glycéryle de stéarate de polyéthylèneglycol (100 OE) | 2,00 g |
| Alcool stéarylique | 1,00 g |
| Huiles | 10,00 g |
| Butylparaben | 0,15 g |
| Méthoxycinnamate d'éthylhexyle | 1,00 g |
| *Phase B* | |
| Conservateurs | 0,50 g |
| EDTA disodique | 0,05 g |
| Poly(acide 2-acrylamido 2-méthylpropane sulfonique) réticulé (HOSTACERIN de HOECHST) | 0,40 g |
| Eau | 63,20 g |

(suite)

| | |
|---|---|
| *Phase* C | |
| Gomme de xanthane | 0,20 g |
| *Phase D* | |
| Mélange de polyuréthane aliphatique, N-méthyl pyrrolidone et diméthyl éthanolamine dans l'eau (AVALURE UR410 de NOVEON) | 20,00 g |

La composition ci-dessus peut être préparée de la façon suivante. Les constituants de la phase B sont chauffés à 75°C environ, sauf le poly(acide 2-acrylamido 2-méthylpropane sulfonique) réticulé qui n'est introduit qu'à cette température. Après agitation jusqu'à l'obtention d'un gel homogène, la phase A chauffée à 75°C environ est incorporée dans la phase B. La phase C est ensuite ajoutée à cette émulsion, sous agitation, puis la phase D à 40-45°C. L'agitation est maintenue jusqu'à refroidissement complet.

**Exemple 2 : composition cosmétique**

| | |
|---|---|
| *Phase* A | |
| Mélange d'isostéaryle de polyglycéryle (4 moles), laurate d'hexyle et poly méthylcétyl diméthyl méthylsiloxane oxyéthyléné oxypropyléné (ABIL WE09 de GOLDSCHMIDT) | 0,79 g |
| Cyclopentasiloxane | 7,21 g |
| Polydiméthylsiloxane | 0,90 g |
| Huile d'amande d'abricot | 4,00 g |
| *Phase B* | |
| Eau | 68,15 g |
| Phénoxyéthanol | 1,00 g |
| Sel pentasodique d'éthylène diamine tétraméthylène phosphonate Copolymère acide acrylique/méthacrylate de stéaryle | 0,05 g |
| (PEMULEN TR1 de NOVEON) | 0,60 g |
| Hydroxyde de sodium | 0,30 g |
| *Phase C* | |
| Mélange de polyuréthane aliphatique, N-méthyl pyrrolidone et diméthyl éthanolamine dans l'eau (AVALURE UR410 de NOVEON) | 17,00 g |

La composition ci-dessus peut être préparée de la façon suivante. Les constituants de la phase B sont chauffés à 75°C environ, sauf le copolymère acrylique qui n'est introduit qu'à cette température. Après agitation jusqu'à l'obtention d'un gel homogène, la phase A chauffée à 75°C environ est incorporée dans la phase B. La phase C est ensuite ajoutée à cette émulsion, sous agitation, à 40-45°C. L'agitation est maintenue jusqu'à refroidissement complet.

**Exemple 3 : Composition cosmétique**

| Émulsion multiple: | |
|---|---|
| *Émulsion primaire (A)* | *22,50 g* |
| Cyclopentasiloxane | 3,50 g |
| Huile d'amande d'abricot | 4,00 g |
| Eau | 51,05 g |
| Phénoxyéthanol : | 1,00 g |
| Sel pentasodique d'éthylène diamine tétraméthylène phosphonate | 0,05 g |
| Copolymère acide acrylique / méthacrylate de stéaryle (PEMULEN TR1 de NOVEON) | 0,60 g |
| Hydroxyde de sodium | 0,30 g |
| Mélange de polyuréthane aliphatique, N-méthyl pyrrolidone et diméthyl éthanolamine dans l'eau (AVALURE UR410 de NOVEON) | 17,00 g |

(suite)

| *Émulsion primaire (A):* | |
|---|---|
| Eau : | 75,20 g |
| Mélange d'isostéaryle de polyglycéryle (4 moles), laurate d'hexyle et poly méthylcétyl diméthyl méthylsiloxane oxyéthyléné oxypropyléné (ABIL WE09 de GOLDSCHMIDT) | 3,50 g |
| Cyclopentasiloxane | 16,50 g |
| Polydiméthylsiloxane | 4,00 g |
| Sulfate de magnésium | 0,80 g |

Mode Opératoire

*Préparation de l'émulsion primaire :*

A température ambiante et sous agitation, on homogénéise l'ABIL WE09, le cyclopentasiloxane et le polydiméthylsiloxane. Sous forte agitation, on incorpore lentement l'eau et le sulfate de magnésium.

*Préparation de l'émulsion multiple :*

A température ambiante et sous agitation, on disperse le copolymère acrylique, le phénoxyéthanol et le séquestrant phosphonique. On laisser gonfler environ 45 minutes sous agitation, puis on ajoute l'AVALURE UR410. On neutralise ensuite avec l'hydroxyde de sodium, puis on dilue l'émulsion primaire avec le cyclopentasiloxane et l'huile d'amande d'abricot. On incorpore alors ce mélange lentement sous agitation à la phase aqueuse.

**Exemple 4 : Composition cosmétique**

| | |
|---|---|
| *Phase A* | |
| Eau | 35,00 g |
| Sulfopolyester EASTMAN AQ55S de EASTMAN CHEMICAL | 2,00 g |
| Conservateurs | 1,00 g |
| *Phase B* | |
| Cyclopentasiloxane | 20,00 g |
| *Phase C* | |
| Gomme de xanthane | 0,50 g |
| Eau | 21,50 g |
| *Phase D* | |
| Mélange de polyuréthane aliphatique, N-méthyl pyrrolidone et diméthyl éthanolamine dans l'eau (AVALURE UR410 de NOVEON) | 20,00 g |

La composition ci-dessus a été préparée de la façon suivante. On a mélangé les constituants de la phase A et chauffé le mélange à 70°C sous agitation magnétique jusqu'à dispersion complète du polymère, puis on a a refroidi la solution jusqu'à température ambiante. Les phases B et C ont été préparées séparément (à chaud ?). La phase A a ensuite été introduite dans la phase B sous vive agitation, puis la phase C. L'émulsion a été homogénéisée sous une pression de 600 bars (2 à 4 passages) en ramenant l'émulsion à température ambiante entre chaque passage. La phase D a alors été ajoutée à l'émulsion.

**Exemple 5 : Mise en évidence de l'effet tenseur**

a) Protocole

Le pouvoir tenseur des compositions cosmétiques des Exemples 1 à 4 a été mesuré à l'extensomètre (MTT 610 fourni par la société DIA-STRON). Le principe de la méthode consiste à mesurer la longueur d'une éprouvette de stratum corneum isolé à partir de peau humaine provenant d'une opération chirurgicale, avant et après traitement avec

les compositions à tester.

Pour ce faire, l'éprouvette est placée entre les deux mâchoires de l'appareil dont l'une est fixe et l'autre mobile, dans une atmosphère à 30°C et 40% d'humidité relative. On exerce une traction sur l'éprouvette, et on enregistre la courbe de la force (en grammes) en fonction de la longueur (en millimètres), la longueur zéro correspondant au contact entre les deux mors de l'appareil. On trace ensuite la tangente à la courbe dans sa région linéaire. L'intersection de cette tangente avec l'axe des abscisses correspond à la longueur apparente $L_0$ de l'éprouvette à force nulle. On détend ensuite l'éprouvette puis on applique sur le stratum corneum 2 mg/cm$^2$ de la composition à tester. Après 15 minutes de séchage, les étapes ci-dessus sont à nouveau mises en oeuvre pour déterminer la longueur $L_1$ de l'éprouvette après traitement.

Le pourcentage de rétractation est défini par :

$$\% \text{ rétractation} = 100 \times (L_1-L_0)/L_0.$$

Pour caractériser un effet tenseur, ce pourcentage doit être négatif et l'effet tenseur est d'autant plus important que la valeur absolue du pourcentage de rétractation est élevée. Le pouvoir tenseur des compositions décrites dans la présente demande de brevet est caractérisé par une rétraction du stratum corneum isolé de plus de 1% en valeur absolue à 30°C sous une humidité relative de 40% (voir tableau 1).

b) Résultats

Les résultats obtenus sont rassemblés dans le Tableau 1 suivant.

Tableau 1 Rétraction du *stratum corneum* isolé

| **Compositions** | **% de Rétractation d'une éprouvette de stratum corneum isolé** |
|---|---|
| Exemple 1 : Émulsion directe H/E à 7% d'Avalure UR410 *(teneur en tensioactif : 3,5%).* | -0.57% +/- 0.26 |
| Exemple 2 : Émulsion directe H/E à 6% d'Avalure UR410. *(teneur en tensioactif : 0,8%).* | -1.56% +/- 0.31 |
| Exemple 3 : Émulsion multiple E/H/E à 6% d'Avalure UR410 *(tenseur en tensioactif : 0,8%)* | -1.50% +/- 0.35 |
| Exemple 4 : Emulsion H/E à 7% d'Avalure UR410 *(pas de tensioactif)* | -2.76% +/- 0.77 |

Comme il ressort de ce tableau, l'effet tenseur d'une émulsion H/E riche en tensioactifs et contenant 7% d'Avalure UR 410 n'est pas significative (Exemple comparatif 1). Au contraire, les émulsions H/E et E/H/E selon l'invention (Exemples 2 à 4) contenant 6% ou 7% d'Avalure UR410 et moins de 1% de tensioactif produisent un effet tenseur permettant d'envisager leur utilisation pour retendre la peau et lisser les rides et ridules.

**Exemple 6 : Évaluation sensorielle**

La composition de l'Exemple 3 a été testée sur un panel de femmes âgées de 40 à 60 ans présentant des rides et ridules au niveau du contour de l'oeil. Il a été observé un effet de lissage des ridules se trouvant sous l'oeil.

**Exemple 7 : Test de stabilité**

On a évalué la stabilité dans le temps et en température des compositions des Exemples 1 à 4 ci-dessus : il n'a été observé aucune modification physico-chimique de ces compositions pendant deux mois à 4°C, 25°C, 37°C et 45°C, en ce sens que leur pH, leur viscosité (mesurée avec un rhéomètre Rhéomat RM180 de la société METTLER), leur aspect et leur odeur sont restés inchangés.

**Revendications**

1. Composition adaptée à une application topique sur la peau et renfermant une phase aqueuse, une phase grasse et une dispersion de particules de polymère produisant, à une concentration de 7% en poids dans l'eau, une rétraction du *stratum corneum* isolé de plus de 1,5% à 30°C et sous une humidité relative de 40%, **caractérisée en ce que** ladite composition renferme au moins un polymère amphiphile ionique et moins de 1 % en poids, par rapport au poids total de la composition, de tensioactif différent dudit polymère amphiphile ionique.

2. Composition selon la revendication 1, **caractérisée en ce que** ladite dispersion de particules de polymère comprend au moins un polymère choisi parmi : les polymères et copolymères de polyuréthanne, les polymères et copolymères acryliques ; les polymères d'acide isophtalique sulfoné ; et les polymères siliconés greffés.

3. Composition selon la revendication 2, **caractérisée en ce que** ladite dispersion de particules de polymère comprend au moins une dispersion de particules de polyester-polyuréthanne.

4. Composition selon la revendication 2, **caractérisée en ce que** ledit polymère siliconé greffé est un polydiméthylsiloxane sur lequel sont greffés, par l'intermédiaire d'un chaînon de raccordement de type thiopropylène, des motifs polymères mixtes du type acide poly(méth)acrylique et du type poly(méth)acrylate d'alkyle.

5. Composition selon la revendication 4, **caractérisée en ce que** ledit polymère siliconé greffé est un copolymère de polydiméthylsiloxane à groupements propylthio, d'acrylate de méthyle, de méthacrylate de méthyle et d'acide méthacrylique.

6. Composition selon la revendication 2, **caractérisée en ce que** ledit polymère synthétique est choisi parmi les réseaux de polymères interpénétrés.

7. Composition selon la revendication 6, **caractérisée en ce que** ledit polymère inerpénétré se trouve sous la forme d'une dispersion aqueuse de particules à base de polyuréthane et polyacrylique, ayant une taille moyenne, en poids, comprise entre 90 et 110 nm, une taille moyenne, en nombre, d'environ 80 nm et une température de transition vitreuse, Tg, qui va d'environ -60°C à +100°C.

8. Composition selon l'une quelconque des revendications 1 à 7, **caractérisée en ce que** ledit polymère amphiphile est choisi parmi : les copolymères acryliques, les dérivés d'acide 2-acrylamido 2-méthylpropane sulfonique hydrophobés, les terpolymères acryliques, et les copolyesters sulfo-isophtaliques.

9. Composition selon la revendication 8, **caractérisée en ce que** les copolymères acryliques sont obtenus par copolymérisation d'un monomère (a) choisi parmi les acides carboxyliques à insaturation $\alpha,\beta$-éthylénique avec un monomère (b) issu de la réaction d'un monomère acide carboxylique à insaturation éthylénique avec un alcool gras aliphatique éventuellement alcoxylé, en particulier polyéthoxylé, dont la chaîne carbonée renferme au moins 6 atomes de carbone.

10. Composition selon la revendication 9, **caractérisée en ce que** les copolymères acryliques sont constitués de polymères formés à partir d'un mélange de monomères comprenant de l'acide acrylique et un ester de formule (III) :

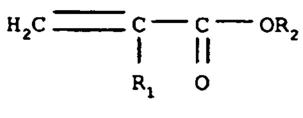

**(III)**

dans laquelle $R_1$ désigne H ou $CH_3$, $R_2$ désignant un radical alkyle ayant de 12 à 22 atomes de carbone, et un agent réticulant.

11. Composition selon la revendication 9, **caractérisée en ce que** les copolymères acryliques sont constitués de 95 à 60% en poids d'acide acrylique, 4 à 40% en poids d'acrylate d'alkyles en $C_{10}$-$C_{30}$, et 0 à 6% en poids de monomère polymérisable réticulant, ou 98 à 96% en poids d'acide acrylique, 1 à 4% en poids d'acrylate d'alkyles en $C_{10}$-$C_{30}$, et 0,1 à 0,6% en poids de monomère polymérisable réticulant.

**12.** Composition selon la revendication 11, **caractérisée en ce que** le monomère polymérisable réticulant est choisi parmi : le polyallylsucrose et le polyallylpentaérythritol.

**13.** Composition selon la revendication 9, **caractérisée en ce que** le copolymère acrylique est obtenu par copolymérisation d'un monomère (a) choisi parmi les acides carboxyliques à insaturation $\alpha,\beta$-éthylénique avec un monomère (b) issu de la réaction de l'acide itaconique avec un alcool gras aliphatique alcoxylé, en particulier polyéthoxylé, comprenant de 1 à 50 motifs oxyde d'alkylène, dont la chaîne carbonée renferme au moins 6 atomes de carbone.

**14.** Composition selon la revendication 8, **caractérisée en ce que** le dérivé d'acide 2-acrylamido 2-méthylpropane sulfonique hydrophobé est choisi parmi les polymères amphiphiles réticulés ou non d'acide 2-acrylamido 2-méthylpropane sulfonique et d'au moins un monomère à insaturation éthylénique comportant au moins une partie hydrophobe ayant de 6 à 30 atomes de carbone.

**15.** Composition selon la revendication 14, **caractérisée en ce que** le dérivé d'acide 2-acrylamido 2-méthylpropane sulfonique hydrophobé est un copolymère constitué :

(a) de 40 à 99% en moles de motif acide 2-acrylamido 2-méthylpropane sulfonique de formule (IV) :

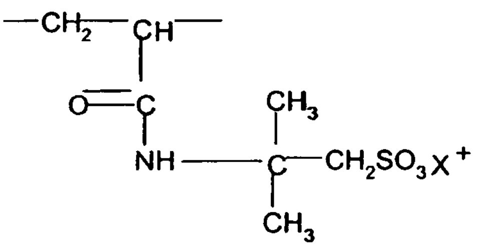
(IV)

dans laquelle $X^+$ est un proton, un cation de métal alcalin, un cation alcalino-terreux ou l'ion ammonium ; et
(b) de 1 à 60% en moles de motif de formule (V) :

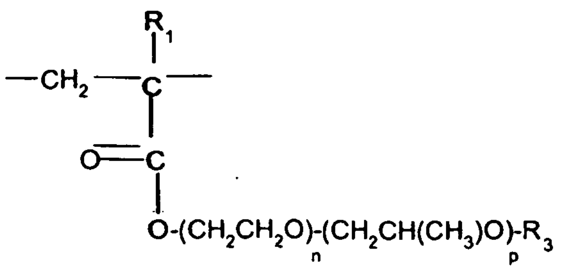
(V)

dans laquelle n et p, indépendamment l'un de l'autre désignent un nombre de moles et varie de 0 à 30, sous réserve que n + p soit inférieur ou égal à 30 ; $R_1$ désigne un atome d'hydrogène ou un radical alkyle linéaire ou ramifié en $C_1$-$C_6$ et $R_3$ désigne un alkyle linéaire ou ramifié comportant de 6 à 30 atomes de carbone.

**16.** Composition selon la revendication 8, **caractérisée en ce que**le terpolymère acrylique est obtenu à partir de (a) un acide carboxylique à insaturation a,a-éthylénique, (b) un monomère à insaturation éthylénique non tensioactif différent de (a), et (c) un monomère uréthanne non-ionique qui est le produit de réaction d'un composé amphiphile non-ionique monohydrique avec un isocyanate à insaturation monoéthylénique.

**17.** Composition selon la revendication 16, **caractérisée en ce que**le terpolymère acrylique est un terpolymère acide méthacrylique/acrylate de méthyle/diméthyl m-isopropénylbenzylisocyanate d'alcool béhénylique éthoxylé à 40 OE.

**18.** Composition selon la revendication 8, **caractérisée en ce que** le terpolymère acrylique est formé à partir de (a) 20 à 60% en poids d'acide acrylique et/ou d'acide méthacrylique, (b) de 5 à 60% en poids de (méth)acrylates d'alkyles inférieurs, de 2 à 50% en poids d'éther d'allyle à chaîne grasse de formule (VI)

CH2 = C(R') CH2 O Bn R (VI)

dans laquelle R' désigne H ou $CH_3$, B désigne le radical éthylèneoxy, n est nul ou désigne un entier allant de 1 à 100, R désigne un radical hydrocarboné choisi parmi les radicaux alkyl, arylalkyl, aryl, alkylaryl, cycloalkyl, comprenant 8 à 30 atomes de carbone, et (c) de 0 à 1% en poids d'un agent réticulant qui est un monomère insaturé polyéthylénique copolymérisable choisi parmi le phtalate de diallyle, le (méth)acrylate d'allyle, le divinylbenzène, le diméthacrylate de (poly)éthylèneglycol, et le méthylène-bis-acrylamide.

**19.** Composition selon la revendication 18, **caractérisée en ce que** le terpolymère acrylique est un terpolymère réticulé d'acide méthacrylique, d'acrylate d'éthyle, d'allyléther de polyéthylèneglycol (10 OE) éther d'alcool stéarylique (40/50/10).

**20.** Composition selon la revendication 8, **caractérisée en ce que** ledit copolyester sulfo-isophtalique consiste essentiellement en des unités répétées d'acide isophtalique ou d'esters ou de chlorure d'acide isophtalique, de diol et d'acide sulfo-isophtalique.

**21.** Composition selon la revendication 20, **caractérisée en ce que** ledit copolyester sulfoisophtalique comprend, pour 100 moles d'acides, de 75 % à 90 % en mole d'acide isophtalique et de 10 % à 25 % en mole d'acide sulpho-isophtalique, notamment de sel de sodium de l'acide 5-sulfoisophtalique, et, pour 100 moles de diols, de 45 % à 85 % en mole de diéthylène glycol et de 15 % à 55 % en mole de 1,4-cyclohexanediméthanol ou d'éthylène glycol ou leur mélange.

**22.** Composition selon l'une quelconque des revendications 1 à 21, **caractérisée en ce qu'**elle renferme de 0,5 à 2% en poids de polymère amphiphile ionique, par rapport au poids total de la composition.

**23.** Composition selon l'une quelconque des revendications précédentes, **caractérisée en ce qu'**elle renferme au moins 45% en poids d'eau.

**24.** Composition selon l'une quelconque des revendications précédentes, **caractérisée en ce qu'**elle renferme au moins 1% en poids de phase grasse.

**25.** Procédé de traitement cosmétique d'une peau ridée, comprenant l'application sur ladite peau de la composition selon l'une quelconque des revendications 1 à 24.

**26.** Utilisation cosmétique de la composition selon l'une quelconque des revendications 1 à 24 pour lisser ou atténuer les rides et ridules et/ou retendre la peau.

## Claims

**1.** Composition that is suitable for topical application to the skin, containing an aqueous phase, a fatty phase and a dispersion of polymer particles that produce, at a concentration of 7% by weight in water, a retraction of isolated *stratum corneum* of more than 1.5% at 30°C and under a relative humidity of 40%, **characterized in that** the said composition contains at least one ionic amphiphilic polymer and less than 1% by weight, relative to the total weight of the composition, of surfactant other than the said ionic amphiphilic polymer.

**2.** Composition according to Claim 1, **characterized in that** the said polymer particle dispersion comprises at least one polymer chosen from: polyurethane polymers and copolymers, acrylic polymers and copolymers; sulfonated isophthalic acid polymers; and grafted silicone polymers.

**3.** Composition according to Claim 2, **characterized in that** the said polymer particle dispersion comprises at least one dispersion of polyester-polyurethane particles.

**4.** Composition according to Claim 2, **characterized in that** the said grafted silicone polymer is a polydimethylsiloxane onto which are grafted, via a linking chain of thiopropylene type, mixed polymer units of the poly(meth)acrylic acid type and of the polyalkyl (meth)acrylate type.

**5.** Composition according to Claim 4, **characterized in that** the said grafted silicone polymer is a copolymer of polydimethylsiloxane containing propylthio groups, of methyl acrylate, of methyl methacrylate and of methacrylic acid.

**6.** Composition according to Claim 2, **characterized in that** the said synthetic polymer is chosen from interpenetrated polymer networks.

**7.** Composition according to Claim 6, **characterized in that** the said interpenetrated polymer is in the form of an aqueous dispersion of particles based on polyurethane and polyacrylic, with a weight-average size of between 90 and 110 nm, a number-average size of about 80 nm and a glass transition temperature, Tg, ranging from about -60°C to +100°C.

**8.** Composition according to any one of Claims 1 to 7, **characterized in that** the said amphiphilic polymer is chosen from: acrylic copolymers, hydrophobic 2-acrylamido-2-methylpropanesulfonic acid derivatives, acrylic terpolymers and sulfoisophthalic copolyesters.

**9.** Composition according to Claim 8, **characterized in that** the acrylic copolymers are obtained by copolymerization of a monomer (a) chosen from α,β-ethylenically unsaturated carboxylic acids with a monomer (b) derived from the reaction of an ethylenically unsaturated carboxylic acid monomer with an optionally alkoxylated, in particular polyethoxylated, aliphatic fatty alcohol, the carbon-based chain of which contains at least 6 carbon atoms.

**10.** Composition according to Claim 9, **characterized in that** the acrylic copolymers consist of polymers formed from a mixture of monomers comprising acrylic acid and an ester of formula (III):

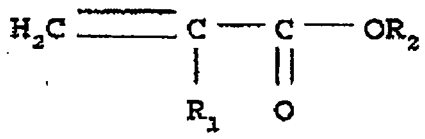

(III)

in which $R_1$ denotes H or $CH_3$, $R_2$ denoting an alkyl radical containing from 12 to 22 carbon atoms, and a crosslinking agent.

**11.** Composition according to Claim 9, **characterized in that** the acrylic copolymers consist of 95% to 60% by weight of acrylic acid, 4% to 40% by weight of $C_{10}$-$C_{30}$ alkyl acrylate and 0% to 6% by weight of crosslinking polymerizable monomer, or 98% to 96% by weight of acrylic acid, 1% to 4% by weight of $C_{10}$-$C_{30}$ alkyl acrylate and 0.1% to 0.6% by weight of crosslinking polymerizable monomer.

**12.** Composition according to Claim 11, **characterized in that** the crosslinking polymerizable monomer is chosen from: polyallylsucrose and polyallylpentaerythritol.

**13.** Composition according to Claim 9, **characterized in that** the acrylic copolymer is obtained by copolymerization of a monomer (a) chosen from α,β-ethylenically unsaturated carboxylic acids with a monomer (b) derived from the reaction of itaconic acid with an alkoxylated, in particular polyethoxylated, aliphatic fatty alcohol comprising from 1 to 50 alkylene oxide units, the carbon-based chain of which contains at least 6 carbon atoms.

**14.** Composition according to Claim 8, **characterized in that** the hydrophobic 2-acrylamido-2-methylpropanesulfonic acid derivative is chosen from crosslinked or non-crosslinked amphiphilic polymers of 2-acrylamido-2-methylpropanesulfonic acid and of at least one ethylenically unsaturated monomer comprising at least one hydrophobic portion containing from 6 to 30 carbon atoms.

**15.** Composition according to Claim 14, **characterized in that** the hydrophobic 2-acrylamido-2-methylpropanesulfonic acid derivative is a copolymer consisting of:

(a) from 40 mol% to 99 mol% of 2-acrylamido-2-methylpropanesulfonic acid units of formula (IV):

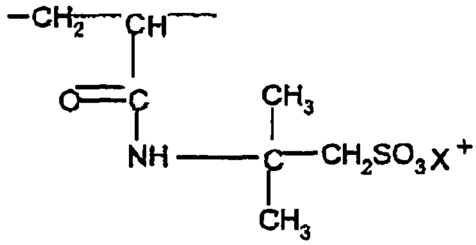

(IV)

in which $X^+$ is a proton, an alkali metal cation, an alkaline-earth metal cation or an ammonium ion; and
(b) from 1 mol% to 60 mol% of units of formula (V):

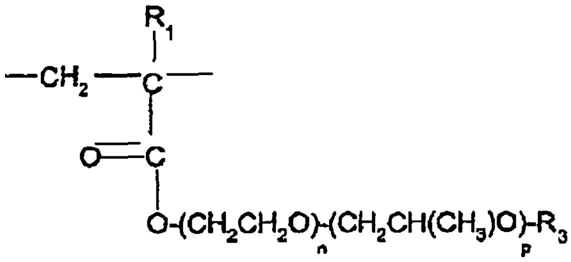

(V)

in which n and p, independently of each other, denote a number of moles and range from 0 to 30, with the proviso that n + p is less than or equal to 30; $R_1$ denotes a hydrogen atom or a $C_1$-$C_6$ linear or branched alkyl radical and $R_3$ denotes a linear or branched alkyl containing from 6 to 30 carbon atoms.

**16.** Composition according to Claim 8, **characterized in that** the acrylic terpolymer is obtained from (a) an α,α-ethylenically unsaturated carboxylic acid, (b) a non-surfactant ethylenically unsaturated monomer different from (a), and (c) a nonionic urethane monomer which is the product of reaction of a monohydric nonionic amphiphilic compound with a monoethylenically unsaturated isocyanate.

**17.** Composition according to Claim 16, **characterized in that** the acrylic terpolymer is a terpolymer of methacrylic acid/ methyl acrylate/dimethyl-m-isopropenylbenzylisocyanate of behenyl alcohol ethoxylated with 40 EO.

**18.** Composition according to Claim 8, **characterized in that** the acrylic terpolymer is formed from (a) 20% to 60% by weight of acrylic acid and/or of methacrylic acid, (b) from 5% to 60% by weight of lower alkyl (meth)acrylates, from 2% to 50% by weight of fatty-chain allyl ether of formula (VI)

$$CH_2 = C(R')CH_2OB_nR \qquad (VI)$$

in which R' denotes H or $CH_3$, B denotes an ethyleneoxy radical, n is zero or denotes an integer ranging from 1 to 100, R denotes a hydrocarbon-based radical chosen from alkyl, arylalkyl, aryl, alkylaryl and cycloalkyl radicals, containing from 8 to 30 carbon atoms, and (c) from 0% to 1% by weight of a crosslinking agent which is a copolymerizable polyethylenically unsaturated monomer chosen from diallyl phthalate, allyl (meth) acrylate, divinylbenzene, (poly)ethylene glycol dimethacrylate and methylenebisacrylamide.

**19.** Composition according to Claim 18, **characterized in that** the acrylic terpolymer is a crosslinked terpolymer of methacrylic acid, of ethyl acrylate and of stearyl alcohol ether of polyethylene glycol allyl ether (10 EO) (40/50/10).

**20.** Composition according to Claim 8, **characterized in that** the said sulfoisophthalic copolyester consists essentially of repeated units of isophthalic acid or of isophthalic acid esters or of isophthalic acid chloride, of diol and of sulfoisophthalic acid.

**21.** Composition according to Claim 20, **characterized in that** the said sulfoisophthalic copolyester comprises, per 100 mol of acids, from 75 mol% to 90 mol% of isophthalic acid and from 10 mol% to 25 mol% of sulfoisophthalic acid, especially of sodium salt of 5-sulfoisophthalic acid, and, per 100 mol of diols, from 45 mol% to 85 mol% of diethylene glycol and from 15 mol% to 55 mol% of 1,4-cyclohexanedimethanol or of ethylene glycol, or a mixture thereof.

22. Composition according to any one of Claims 1 to 21, **characterized in that** it contains from 0.5% to 2% by weight of ionic amphiphilic polymer relative to the total weight of the composition.

23. Composition according to any one of the preceding claims, **characterized in that** it contains at least 4.5% by weight of water.

24. Composition according to any one of the preceding claims, **characterized in that** it contains at least 1% by weight of fatty phase.

25. Cosmetic process for treating wrinkled skin, comprising the application of the composition according to any one of Claims 1 to 24 to the said skin.

26. Cosmetic use of the composition according to any one of Claims 1 to 24, to smooth out or attenuate wrinkles and fine lines and/or to restore the skin's tautness.

**Patentansprüche**

1. Zusammensetzung, die für eine topische Anwendung auf die Haut geeignet ist und die eine wässrige Phase, eine Fettphase und eine Dispersion von Polymerpartikeln enthält, die in einer Konzentration von 7 Gew.-% in Wasser bei 30 °C und einer relativen Luftfeuchte von 40 % eine Retraktion von isoliertem *Stratum corneum* von mehr als 1,5 % hervorrufen, **dadurch gekennzeichnet, dass** die Zusammensetzung ferner mindestens ein ionisches amphiphiles Polymer und mindestens 1 Gew.-% eines grenzflächenaktiven Stoffes, der von dem ionischen amphiphilen Polymer verschieden ist, bezogen auf das Gesamtgewicht der Zusammensetzung, enthält.

2. Zusammensetzung nach Anspruch 1, **dadurch gekennzeichnet, dass** die Dispersion von Polymerpartikeln mindestens ein Polymer umfasst, das ausgewählt ist unter: Polymeren und Copolymeren von Polyurethan, Acrylpolymeren und Acrylcopolymeren; Polymeren von sulfonierter Isophthalsäure; und gepfropften Siliconpolymeren.

3. Zusammensetzung nach Anspruch 3, **dadurch gekennzeichnet, dass** die Dispersion von Polymerpartikeln mindestens eine Dispersion von Polyesterpolyurethanpartikeln umfasst.

4. Zusammensetzung nach Anspruch 2, **dadurch gekennzeichnet, dass** das gepfropfte Siliconpolymer ein Polydimethylsiloxan ist, auf das über eine Verbindungsgruppe vom Thiopropylentyp gemischte Polymereinheiten vom Typ Poly(meth)acrylsäure und Polyalkyl(meth)acrylat gepfropft sind.

5. Zusammensetzung nach Anspruch 4, **dadurch gekennzeichnet, dass** das gepfropfte Siliconpolymer ein Copolymer von Polydimethylsiloxan mit Propylthiogruppen, Methylacrylat, Methylmethacrylat und Methacrylsäure ist.

6. Zusammensetzung nach Anspruch 2, **dadurch gekennzeichnet, dass** das synthetische Polymer unter den interpenetrierenden Polymernetzen ausgewählt ist.

7. Zusammensetzung nach Anspruch 6, **dadurch gekennzeichnet, dass** das interpenetrierende Polymer in Form einer wässrigen Dispersion von Partikeln auf Polyurethanbasis und Polyacrylbasis vorliegt, die eine gewichtsmittlere Größe von 90 bis 110 nm und eine zahlenmittlere Größe von etwa 80 nm aufweisen und eine Glasübergangstemperatur Tg besitzen, die im Bereich von etwa -60 bis + 100 °C liegt.

8. Zusammensetzung nach einem der Ansprüche 1 bis 7, **dadurch gekennzeichnet, dass** das amphiphile Polymer ausgewählt ist unter: Acrylcopolymeren, hydrophoben Derivaten von 2-Acrylamido-2-methylpropansulfonsäure, Acrylterpolymeren und Sulfoisophthalsäurecopolyestern.

9. Zusammensetzung nach Anspruch 8, **dadurch gekennzeichnet, dass** die Acrylcopolymere durch Copolymerisation eines Monomers (a), das unter den Carbonsäuren mit $\alpha,\beta$-ethylenisch ungesättigter Bindung ausgewählt ist, und eines Monomers (b) erhalten werden, das bei der Reaktion eines Carbonsäuremonomers mit ethylenisch ungesättigter Bindung und eines gegebenenfalls alkoxylierten und insbesondere ethoxylierten aliphatischen Fettalkohols, dessen Kohlenstoffkette mindestens 6 Kohlenstoffatome aufweist, gebildet wird.

10. Zusammensetzung nach Anspruch 9, **dadurch gekennzeichnet, dass** die Acrylcopolymere aus Polymeren bestehen, die aus einem Gemisch von Monomeren gebildet werden, das Acrylsäure, einen Ester der folgenden Formel (III)

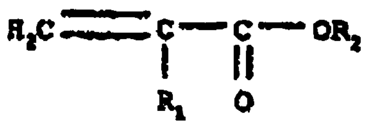

(III),

worin $R_1$ H oder $CH_3$ und $R_2$ eine Alkylgruppe mit 12 bis 22 Kohlenstoffatomen bedeutet, und ein Vernetzungsmittel enthält.

**11.** Zusammensetzung nach Anspruch 9, **dadurch gekennzeichnet, dass** die Acrylcopolymere aus 95 bis 60 Gew.-% Acrylsäure, 4 bis 40 Gew.-% $C_{10-30}$-Alkylacrylat und 0 bis 6 Gew.-% eines vernetzenden polymerisierbaren Monomers oder aus 98 bis 96 Gew.-% Acrylsäure, 1 bis 4 Gew.-% $C_{10-30}$-Alkylacrylat und 0,1 bis 0,6 Gew.-% eines vernetzenden polymerisierbaren Monomers bestehen.

**12.** Zusammensetzung nach Anspruch 11, **dadurch gekennzeichnet, dass** das verrietzende polymerisierbare Monomer unter Polyallylsaccharose und Polyallylpentaerythrit ausgewählt ist.

**13.** Zusammensetzung nach Anspruch 9, **dadurch gekennzeichnet, dass** das Acrylcopolymer durch Copolymerisation eines Monomers (a), das unter den Carbonsäuren mit $\alpha,\beta$-ethylenisch ungesättigter Bindung ausgewählt ist, und einem Monomer (b) hergestellt wird, das bei der Reaktion von Itaconsäure mit einem alkoxylierten und insbesondere polyethoxylierten aliphatischen Fettalkohol mit 1 bis 50 Alkyleneinheiten gebildet wird, dessen Kohlenstoffkette mindestens 6 Kohlenstoffatome aufweist.

**14.** Zusammensetzung nach Anspruch 8, **dadurch gekennzeichnet, dass** das hydrophobierte 2-Acrylamido-2-methylpropansäure-sulfonsäurederivat unter den gegebenenfalls vernetzten amphiphilen Polymeren von 2-Acrylamido-2-methylpropansulfonsäure und mindestens einem Monomer mit ethylenisch ungesättigter Bindung ausgewählt ist, das mindestens einen hydrophoben Bereich mit 6 bis 30 Kohlenstoffatomen aufweist.

**15.** Zusammensetzung nach Anspruch 14, **dadurch gekennzeichnet, dass** das hydrophobierte 2-Acrylamido-2-methylpropansulfonsäurederivat ein Copolymer ist, bestehend aus:

(a) 40 bis 99 Mol-% 2-Acylamido-2-methylpropaxisulfonsäureeinheit der Formel (IV):

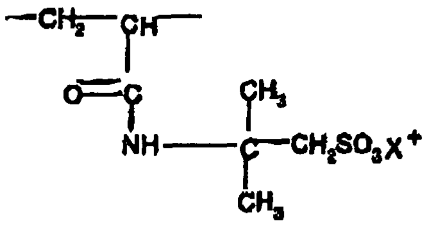

(IV),

worin $X^+$ ein Proton, ein Alkalimetallkation, ein Erdalkalimetallkation oder ein Ammoniumion bedeutet, und
(b) 1 bis 60 Mol-% einer Einheit der Formel (V) :

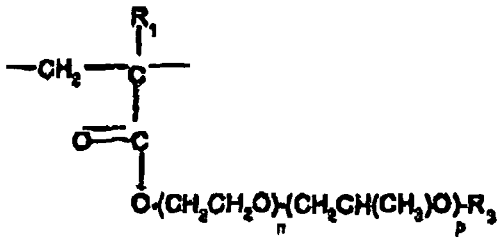

(V),

worin n und p unabhängig voneinander eine Molzahl bedeuten und im Bereich von 0 bis 30 liegen, mit der Maßgabe, dass n + p höchstens 30 beträgt; $R_1$ ein Wasserstoffatom oder eine geradkettige oder verzweigte $C_{1-6}$-Alkylgruppe bedeutet und $R_3$ eine geradkettige oder verzweigte Alkylgruppe mit 6 bis 30 Kohlenstoffatomen ist.

**16.** Zusammensetzung nach Anspruch 8, **dadurch gekennzeichnet, dass** das Acrylterpolymer hergestellt wird aus (a) einer Carbonsäure mit ethylenisch ungesättigter Bindung, (b) einem Monomer mit ethylenisch ungesättigter Bindung, das nicht grenzflächenaktiv und von (a) verschieden ist, und (c) einem nichtionischen Urethanmonomer, bei dem es sich um das Reaktionsprodukt einer nichtionischen amphiphilen Verbindung mit einem Wasserstoffatom und einem Isocyanat mit monoethylenisch ungesättigter Bindung handelt.

**17.** Zusammensetzung nach Anspruch 16, **dadurch gekennzeichnet, dass** das Acrylterpolymer ein Terpolymer Methacrylsäure/Methylacrylat/Dimethyl-m-Isopropenylbenzylisocyanat von ethoxyliertem Behenylalkohol mit 40 EO ist.

**18.** Zusammensetzung nach Anspruch 8, **dadurch gekennzeichnet, dass** das Acrylterpolymer gebildet wird aus (a) 20 bis 60 Gew.-% Acrylsäure und/oder Methacrylsäure, (b) 5 bis 60 Gew.-% Alkyl(meth)acrylaten, wobei es sich bei den Alkylgruppen um niedere Alkylgruppen handelt, 2 bis 50 Gew.-% eines Allylethers mit Fettkette der Formel (VI)

$$CH2 = C(R') CH2 O Bn R \qquad (VI),$$

worin R' H oder $CH_3$ bedeutet, B die Ethylenoxygruppe ist, n Null ist oder eine ganze Zahl im Bereich von 1 bis 100 bedeutet, R eine Kohlenwasserstoffgruppe bedeutet, die unter den Gruppen Alkyl, Arylalkyl, Aryl, Alkylaryl und Cycloalkyl mit 8 bis 30 Kohlenstoffatomen ausgewählt ist, und (c) 0 bis 1 Gew.-% eines Vernetzungsmittels, bei dem es sich um ein polyethylenisch ungesättigtes copolymerisierbares Monomer handelt, das unter Diallylphthalat, Allyl(meth)acrylat, Divinylbenzol, (Poly)ethylenglycoldimethacrylat und Methylen-bis-acrylamid ausgewählt ist.

**19.** Zusammensetzung nach Anspruch 18, **dadurch gekennzeichnet, dass** das Acrylterpolymer ein vernetztes Terpolymer von Methacrylsäure, Ethylacrylat und dem Polyethylenglycol(10 EO)allylether von Stearylalkohol (40/50/10) handelt.

**20.** Zusammensetzung nach Anspruch 8, **dadurch gekennzeichnet, dass** der Sulfoisophthalsäurecopolyester im Wesentlichen aus Wiederholungseinheiten von Isophthalsäure oder Isophthalsäureestern oder Isophthalsäurechlorid, einem Diol und Sulfoisophthalsäure handelt.

**21.** Zusammensetzung nach Anspruch 20, **dadurch gekennzeichnet, dass** das Sulfoisophthalsäurecopolyester auf 100 mol Säuren 75 bis 90 Mol-% Isophthalsäure und 10 bis 25 Mol-% Sulfoisophthalsäure, insbesondere das Natriumsalz von 5-Sulfoisophthalsäure, und auf 100 mol Diole 45 bis 85 Mol-% Diethylenglycol und 15 bis 55 Mol-% 1,4-Cyclohexandimefihanol oder Ethylenglycol oder deren Gemisch enthält.

**22.** Zusammensetzung nach einem der Ansprüche 1 bis 21, **dadurch gekennzeichnet, dass** sie 0,5 bis 2 Gew.-% ionisches amphiphiles Polymer, bezogen auf das Gesamtgewicht der Zusammensetzung, enthält.

**23.** Zusammensetzung nach einem der vorhergehenden Ansprüche, **dadurch gekennzeichnet, dass** sie mindestens 45 Gew.-% Wasser enthält.

**24.** Zusammensetzung nach einem der vorhergehenden Ansprüche, **dadurch gekennzeichnet, dass** sie mindestens 1 Gew.-% Fettphase enthält.

**25.** Verfahren zur kosmetischen Behandlung von Haut, die Falten aufweist, das das Auftragen der Zusammensetzung nach einem der Ansprüche 1 bis 24 auf die Haut umfasst.

**26.** Kosmetische Verwendung der Zusammensetzung nach einem der Ansprüche 1 bis 24, um Falten und/oder Fältchen zu glätten oder abzuschwächen und/oder die Haut zu straffen.